# EUROPEAN PATENT APPLICATION

(11) **EP 4 462 356 A1**
(43) Date of publication of application: **13.11.2024**
(21) Application number: 23845039.9
(22) Date of filing: 26.05.2023
(51) Int. Cl.: G06T 7/00

(54) **PHYSIOLOGICAL IMAGE PROCESSING METHOD AND APPARATUS, MODEL TRAINING METHOD AND APPARATUS, AND DEVICE AND MEDIUM**

(30) Priority: 26.07.2022 CN 202210885202
(71) Applicant: Tencent Technology (Shenzhen) Company Limited, Shenzhen, Guangdong 518057 (CN)
(72) Inventor: TIAN, Kuan, Shenzhen, Guangdong 518057 (CN); ZHANG, Jun, Shenzhen, Guangdong 518057 (CN)
(74) Representative: EP&C
(86) International application number: PCT/CN2023/096574
(87) International publication number: WO 2024/021825

(57) **Abstract**

The present application belongs to the technical field of artificial intelligence. Disclosed are a physiological image processing method and apparatus, a model training method and apparatus, and a device and a medium. The physiological image processing method comprises: acquiring a physiological image that has been subjected to staining processing (510); determining location information of at least one mutated object in the physiological image (520); performing color channel decomposition on the physiological image to obtain staining information corresponding to the physiological image (530); and compiling statistics according to the location information and the staining information, so as to obtain a staining counting result of the mutated object (540). In the present application, a staining counting result of the mutated object is obtained by means of the location information and staining information of the mutated object, such that statistics can be compiled quickly to obtain a staining counting result of the mutated object; and the generalization capability of the physiological image processing method is improved, such that a good counting effect can be obtained for all physiological images that are subjected to staining processing in different ways, the effect of different staining processing ways on physiological images is avoided, and the accuracy of a staining counting result is guaranteed.

## Description

### RELATED APPLICATION

The present disclosure claims priority to Chinese Patent Application No. 202210885202.3, entitled "PHYSIOLOGICAL IMAGE PROCESSING METHOD, MODEL TRAINING METHOD AND APPARATUS, DEVICE AND MEDIUM" filed on July 26, 2022, which is incorporated by reference in its entirety.

### FIELD OF THE TECHNOLOGY

The present disclosure relates to the field of artificial intelligence, and in particular to a physiological image processing method, a model training method and apparatus, a device and a medium.

### BACKGROUND OF THE DISCLOSURE

A physiological image is an image of a physiological section that has been stained, and different staining reagents have different staining effects on cells of different function types.

In the related art, in order to fully observe the composition of different tissues in the physiological section, it is usually required to use multiple staining manners to respectively stain the physiological section. Biological structure information in the physiological image corresponding to the physiological section directly reflects microscopic morphology of the cells in the physiological section. The cells are divided into diseased cells and normal cells.

However, there are often a huge number of diseased cells in the physiological image, so it is an urgent problem to quickly and accurately count the number of diseased cells.

### SUMMARY

The present disclosure provides a physiological image processing method, a model training method and apparatus, a device and a medium. The technical solution is as follows:

According to one aspect of the present disclosure, provides a physiological image processing method is provided. The method is executable by a computer device and includes:
obtaining a physiological image that has been stained;
determining position information of at least one mutated object in the physiological image;
performing color channel decomposition on the physiological image to obtain staining information corresponding to the physiological image; and
counting, according to the position information of the at least one mutated object and the staining information, stained mutated objects in the physiological image to obtain a counting result of the stained mutated objects.

Another aspect of the present disclosure provides a training method of a physiological image processing model. The physiological image processing model includes a detection network. The method includes:
obtaining a sample physiological image and mark information of the sample physiological image;
calling the detection network in the physiological image processing model to perform position detection on the sample physiological image to obtain a predicted detection result of the sample physiological image; and
training the physiological image processing model according to an error between the predicted detection result and the mark information to obtain a trained physiological image processing model.

Another aspect of the present disclosure provides a physiological image processing apparatus, including:
an obtaining module, configured to obtain a physiological image that has been stained;
a determination module, configured to determine position information of at least one mutated object in the physiological image,
the determination module being further configured to perform color channel decomposition on the physiological image to obtain staining information corresponding to the physiological image; and
a counting module, configured to count, according to the position information of the at least one mutated object and the staining information, stained mutated objects in the physiological image to obtain a counting result of the stained mutated objects.

Another aspect of the present disclosure provides a training apparatus of a physiological image processing model. The physiological image processing model includes a detection network. The apparatus includes:
an obtaining module, configured to obtain a sample physiological image and mark information of the sample physiological image;
a prediction module, configured to call the detection network in the physiological image processing model to perform position detection on the sample physiological image to obtain a predicted detection result of the sample physiological image; and
a training module, configured to train the physiological image processing model according to an error between the predicted detection result and the mark information to obtain a trained physiological image processing model.

Another aspect of the present disclosure provides a computer device, including a processor and a memory. The memory stores at least one program, and the processor is configured to execute the at least one program in the memory to implement the physiological image processing method and/or the training method of a physiological image processing model as described in the above aspects.

Another aspect of the present disclosure provides a computer-readable storage medium. The readable storage medium stores at least one program, and the at least one program is loaded and executed by a processor to implement the physiological image processing method and/or the training method of a physiological image processing model as described in the above aspects.

Another aspect of the present disclosure provides a computer device, including computer instructions stored in a computer-readable storage medium. A processor reads and executes the computer instructions from the computer-readable storage medium to implement the physiological image processing method and/or the training method of a physiological image processing model as described in the above aspects.

The beneficial effects of the technical solution provided by the present disclosure at least include:
By obtaining the counting result of the stained mutated objects according to the position information of the mutated objects and the staining information, the counting result of the stained mutated objects in the physiological image is quickly obtained. By respectively performing the position detection and the color channel decomposition on the physiological image to determine the position information of the mutated objects and the staining information, generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

### BRIEF DESCRIPTION OF THE DRAWINGS

FIG. 1 is a schematic diagram of a computer system provided by some exemplary embodiments of the present disclosure.
FIG. 2 is a schematic diagram of a physiological image processing model provided by some exemplary embodiments of the present disclosure.
FIG. 3 is a flow chart of a physiological image processing method provided by some exemplary embodiments of the present disclosure.
FIG. 4 is a flow chart of a physiological image processing method provided by some exemplary embodiments of the present disclosure.
FIG. 5 is a flow chart of a physiological image processing method provided by some exemplary embodiments of the present disclosure.
FIG. 6 is a schematic diagram of a physiological image and a position mark image of physiological objects provided by some exemplary embodiments of the present disclosure.
FIG. 7 is a schematic diagram of a segmentation image and a diseased region mark image of the physiological image provided by some exemplary embodiments of the present disclosure.
FIG. 8 is a schematic diagram of a position mark image of mutated objects provided by some exemplary embodiments of the present disclosure.
FIG. 9 is a flow chart of a physiological image processing method provided by some exemplary embodiments of the present disclosure.
FIG. 10 is a schematic diagram of a physiological object detection result provided by some exemplary embodiments of the present disclosure.
FIG. 11 is a flow chart of a physiological image processing method provided by some exemplary embodiments of the present disclosure.
FIG. 12 is a schematic diagram of a segmentation result image and a diseased region image of the physiological image provided by some exemplary embodiments of the present disclosure.
FIG. 13 is a flow chart of a physiological image processing method provided by some exemplary embodiments of the present disclosure.
FIG. 14 is a schematic diagram of staining information provided by some exemplary embodiments of the present disclosure.
FIG. 15 is a flow chart of a physiological image processing method provided by some exemplary embodiments of the present disclosure.
FIG. 16 is a flow chart of a physiological image processing method provided by some exemplary embodiments of the present disclosure.
FIG. 17 is a schematic diagram of a staining counting image provided by some exemplary embodiments of the present disclosure.
FIG. 18 is a schematic diagram of physiological images and staining counting images provided by some exemplary embodiments of the present disclosure.
FIG. 19 is a flow chart of a training method of a physiological image processing model provided by some exemplary embodiments of the present disclosure.
FIG. 20 is a flow chart of a training method of a physiological image processing model provided by some exemplary embodiments of the present disclosure.
FIG. 21 is a flow chart of a training method of a physiological image processing model provided by some exemplary embodiments of the present disclosure.
FIG. 22 is a structural block diagram of a physiological image processing apparatus provided by some exemplary embodiments of the present disclosure.
FIG. 23 is a structural block diagram of a training apparatus of a physiological image processing model provided by some exemplary embodiments of the present disclosure.
FIG. 24 is a structural block diagram of a server provided by some exemplary embodiments of the present disclosure.

### DESCRIPTION OF EMBODIMENTS

User information (including but not limited to user equipment information, user personal information, etc.) and data (including but not limited to data for analysis, data stored, data displayed, etc.) involved in the present disclosure are all information and data authorized by users or fully authorized by all parties, and the collection, use and processing of relevant data need to comply with relevant laws, regulations and standards of relevant countries and regions. For example, physiological images and other information involved in the present disclosure are all obtained with full authorization.

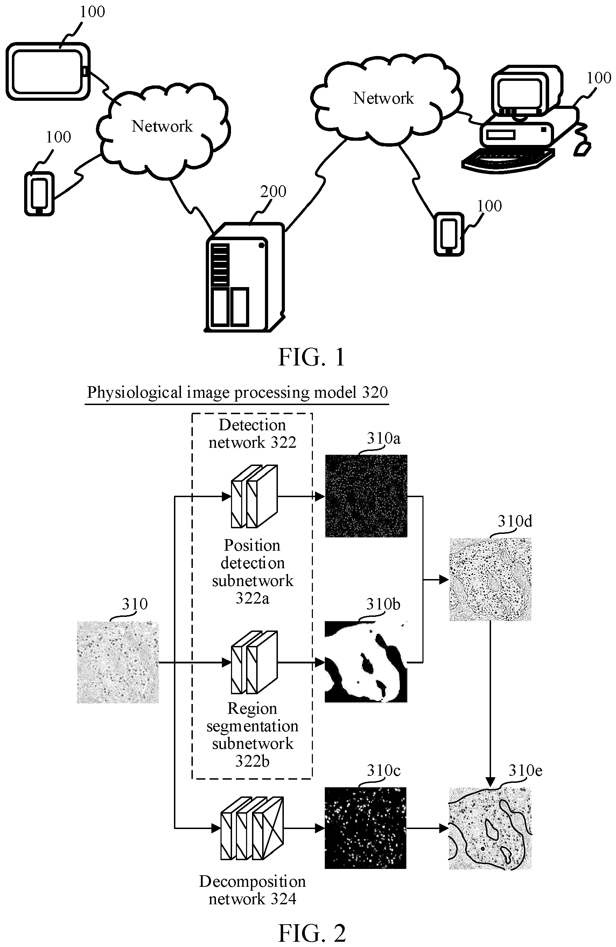

FIG. 1 is a schematic diagram of a computer system provided by an embodiment of the present disclosure. The computer system may be implemented as a system architecture of a physiological image processing method and/or a model training method. The computer system may include: terminals 100 and a server 200. The terminals 100 may be electronic devices such as a mobile phone, a tablet computer, an on board terminal (in-vehicle terminal), a wearable device, a personal computer (PC) and an unmanned reservation terminal. A client running a target application may be installed in the terminal 100. The target application may be an application for training and/or using a physiological image processing model or another application program provided with a training and/or using function of the physiological image processing model, which is not limited in the present disclosure. In addition, the present disclosure does not limit the form of the target application, which includes, but not limited to, an application (App), an applet, a web page, etc. installed in the terminal 100. The server 200 may be an independent physical server, a server cluster or distributed system including a plurality of physical servers, or a cloud server providing cloud computing services. The server 200 may be a background server of the above-mentioned target application, which is configured to provide background services for clients of the target application.

The execution subject of steps of the physiological image processing method and/or the model training method provided by the embodiments of the present disclosure may be a computer device. The computer device refers to an electronic device having data calculation, processing and storage capabilities. In an example of the computer system shown in FIG. 1, the physiological image processing method and/or the model training method may be executed by the terminal 100 (for example, the physiological image processing method and/or the model training method is executed by the client running the target application installed in the terminal 100), or the physiological image processing method and/or the model training method may be executed by the server 200, or by the terminal 100 and the server 200 alternatively and cooperatively, which is not limited in the present disclosure.

In addition, the technical solution of the present disclosure may be combined with the blockchain technology. For example, some data (physiological images, sample physiological images, etc.) involved in the physiological image processing method and/or the model training method disclosed in the present disclosure may be stored in the blockchain. The terminals 100 and the server 200 may communicate through a network, such as a wired or wireless network.

Next, physiological image processing in the present disclosure will be described:

FIG. 2 shows a schematic diagram of a physiological image processing model 320 provided by an embodiment of the present disclosure.

The physiological image processing model 320 is a trained network model. The physiological image processing model 320 includes: a detection network 322 and a decomposition network 324.

A physiological image 310 is obtained. The physiological image 310 is a physiological image that has been stained. Exemplarily, the physiological image 310 is a physiological image obtained by observing a breast tissue section, which is treated by nuclear antigen proliferation index (Ki67) staining.

The physiological image 310 is a digital image obtained by using a charge coupled device (CCD) camera to collect a physiological section placed under a microscope.

The detection network 322 is called to perform position detection on the physiological image. Exemplarily, the detection network 322 includes: a position detection subnetwork 322a and a region segmentation subnetwork 322b.

Specifically, the position detection subnetwork 322a is called to perform the position detection on the physiological image 310 to obtain position information of cells in the physiological image 310. A cell position image 310a shows positions of center points of cells in the physiological image 310. Exemplarily, in this embodiment, physiological objects in the physiological image are cells.

The region segmentation subnetwork 322b is called to perform image segmentation on the physiological image 310 to obtain a diseased region in the physiological image 310. A diseased region image 310b shows the diseased region in the physiological image 310. For example, a diseased region image 310b is a binarized image, the white region in the diseased region image 310b is the diseased region, and the diseased region includes at least one diseased cell. Exemplarily, the diseased cell is a cell with pathological changes in the physiological image, and specifically, the diseased cell belonging to the type of a cancerous cell is also referred to as a cancer cell. In an example, the cause of pathological changes of the cancerous cell is abnormal cell proliferation.

Position information of at least one diseased cell is determined according to the diseased region and the position information of the cells. A diseased position image 3 10d shows positions of center points of diseased cells in the diseased region.

The decomposition network 324 is called to perform color channel decomposition on the physiological image 310 to obtain staining information of the physiological image. Exemplarily, the staining information indicates two staining states of the cells in the physiological image 310: a colored state and an uncolored state. A colored cell image 3 10c shows an image of cells in the colored state. Exemplarily, the cells in the colored state are brown, and the cells in the uncolored state are blue.

According to the positions of the center points of the diseased cells and the staining information, the diseased cells in the colored state are counted to obtain a counting result of the diseased cells in the colored state. A colored diseased cell image 310e shows diseased cells in the colored state and an envelope of the diseased region.

Next, the physiological image processing method will be described through the following embodiments.

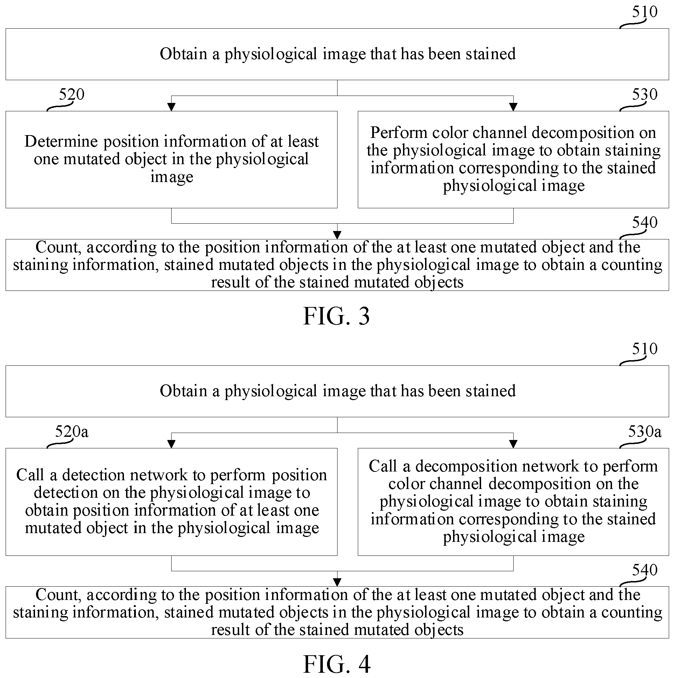

FIG. 3 shows a flow chart of a physiological image processing method provided by some exemplary embodiments of the present disclosure. The method may be executed by a computer device. The method includes:
**Step 510:** Obtain a physiological image that has been stained.

Exemplarily, the physiological image carries microscopic morphology of the physiological object. The physiological image may be obtained from an image of a physiological section, or an image of a physiological tissue that is directly observed, which is not limited in the present disclosure. The physiological object includes, but not limited to, the following one of: cells, tissues, organelles, ribosomes, proteins and antigen receptors.

Exemplarily, the physiological image is a physiological image that has been stained. The physiological section may be stained directly, or the physiological image of the physiological section observed may be stained. In an implementation, the physiological section is stained, and different staining manners have different staining effects on the object.

In an example in which the physiological section is a breast tissue section, the physiological section is stained by immunohistochemistry (IHC) staining. Specifically, nuclear staining in the IHC staining includes, but not limited to, at least one of: estrogen receptor (ER) staining, progesterone receptor (PR) staining, nuclear antigen proliferation index (Ki67) staining, human epidermal growth factor receptor 2 (HER2) staining. Exemplarily, the staining further includes membrane staining.

**Step 520:** Determine position information of at least one mutated object in the physiological image.

Exemplarily, the physiological image includes mutated objects and physiological objects. The mutated object is a physiological object with abnormal changes or pathological changes in the physiological image, or the mutated object is a physiological object with significant distinguishable characteristics in the physiological image. The microstructure of the mutated object is different from the microstructure of the physiological object without pathological changes. The cause of the pathological changes of the mutated object may be destruction of the physiological object, or abnormal proliferation of the physiological object, which is not limited in the present disclosure.

Exemplarily, the position information refers to the position of the mutated objects in the physiological image, i.e., the position information of the mutated objects in an image coordinate system corresponding to the physiological image. The position information of the mutated objects may be indicated by a position information table, or by marking in the physiological image. The present disclosure does not limit the display manner of the position information of the mutated objects. Similarly, the position information of the mutated objects may be determined by determining position information of a plurality of mutated objects one by one, or determining a position region(s) where a plurality of mutated objects are located.

Exemplarily, the position information of the mutated objects may be determined by statistical treatment or neural network model prediction. The manner of determining the position information of the mutated objects is not limited in the present disclosure.

**Step 530:** Perform color channel decomposition on the physiological image to obtain staining information corresponding to the stained physiological image.

Exemplarily, the staining information is used for indicating staining statuses of the stained physiological image, and indicating a staining status of at least one pixel stained in the physiological image.

Exemplarily, the staining information of the stained physiological image may be determined by statistical treatment or neural network model prediction. The manner of determining the staining information of the physiological image is not limited in the present disclosure.

Exemplarily, different staining manners usually have different staining effects on the physiological image, and the physiological images stained by different staining manners usually correspond to different staining information.

**Step 540:** Count, according to the position information and the staining information, stained mutated objects in the physiological image to obtain a counting result of the stained mutated objects.

The counting result of the stained mutated objects is used for indicating a counting result of mutated objects that have been stained. Exemplarily, the counting result of the stained mutated objects may be indicated by staining counting information, or by marking in the physiological image, which is not limited in the present disclosure.

Based on the above, according to the method provided by this embodiment, by obtaining the counting result of the stained mutated objects according to the position information of the mutated objects and the staining information, the counting result of the stained mutated objects in the physiological image is quickly obtained. By respectively performing the position detection and the color channel decomposition on the physiological image to determine the position information of the mutated objects and the staining information, generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

FIG. 4 shows a flow chart of a physiological image processing method provided by some exemplary embodiments of the present disclosure. The method may be executed by a computer device. That is, in the embodiment shown in FIG. 3, step 520 may be implemented as step 520a, and step 530 may be implemented as step 530a:

**Step 520a:** Call the detection network to perform the position detection on the physiological image to obtain the position information of the at least one mutated object in the physiological image.

Exemplarily, the physiological image processing method is executed based on a physiological image processing model. The physiological image processing model includes a detection network and a decomposition network.

The detection network is called to perform the position detection on the physiological image. The detection network includes, but not limited to, at least one of: a convolutional neural network (CNN), a long short-term neural network (LSTM), a recurrent neural network (RNN), fully convolution networks (FCN), a U-Net, a SegNet and a LinkNet.

**Step 530a:** Call the decomposition network to perform the color channel decomposition on the physiological image to obtain the staining information corresponding to the stained physiological image.

Exemplarily, color information carried in the physiological image is decomposed to obtain the staining information corresponding to the stained physiological image. Exemplarily, the decomposition network is called to perform the color channel decomposition on the physiological image according to a color system.

Based on the above, according to the method provided by this embodiment, the physiological image processing method is executed based on the physiological image processing model. By respectively performing the position detection and the color channel decomposition on the physiological image to determine the position information of the mutated objects and the staining information, generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

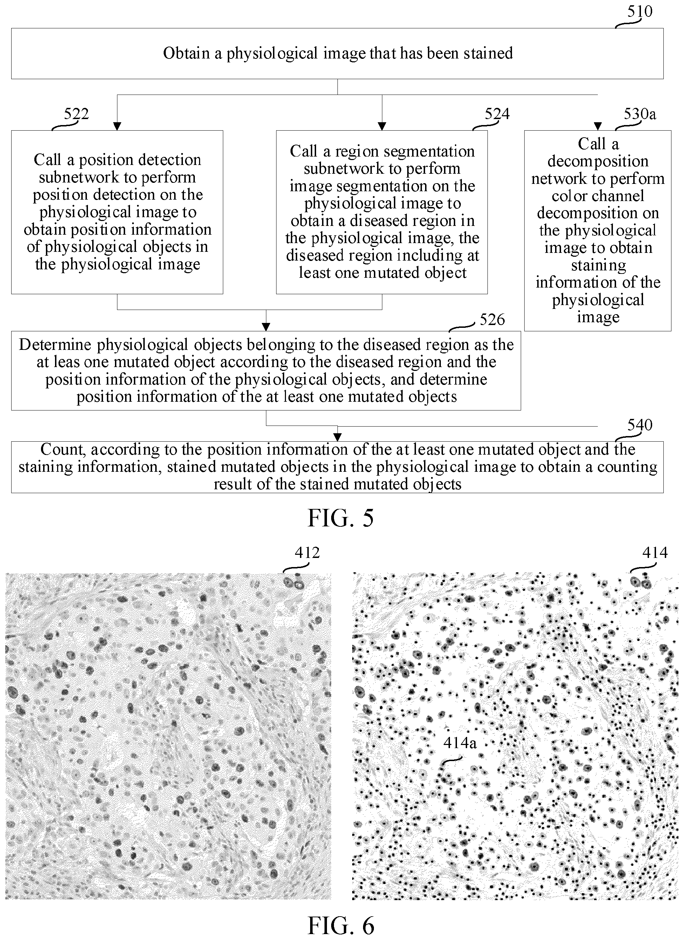

FIG. 5 shows a flow chart of a physiological image processing method provided by some exemplary embodiments of the present disclosure. The method may be executed by a computer device. That is, in the embodiment shown in FIG. 3, step 520a may be implemented as step 522, step 524 and step 526:

**Step 522:** Call a position detection subnetwork to perform the position detection on the physiological image to obtain the position information of physiological objects in the physiological image.

The detection network includes the position detection subnetwork and a region segmentation subnetwork. The position detection subnetwork includes, but not limited to, at least one of: a CNN, an LSTM, an RNN, an FCN, a U-Net, a SegNet and a LinkNet.

Exemplarily, the physiological image includes at least one physiological object, and the physiological object includes the mutated object with pathological changes, and may include another physiological object other than the mutated object, such as a non-mutated object. The present disclosure does not limit the specific type of the physiological object. In an example, the physiological object includes at least one mutated object.

Exemplarily, the position information of the physiological objects may be indicated by a position information table, or by marking in the physiological image. FIG. 6 shows a schematic diagram of a physiological image and a position mark image of the physiological objects. In FIG. 6, the position information of the physiological objects are marked in the physiological image 412 to obtain the position mark image 414 of the physiological objects so as to indicate the position information of the physiological objects. Exemplarily, a marked point 414a in the position mark image 414 of the physiological objects indicates a position of a physiological object. There are a plurality of marked points in the position mark image 414 of the physiological objects that respectively indicate the positions of the plurality of physiological objects.

**Step 524:** Call the region segmentation subnetwork to perform image segmentation on the physiological image to obtain a diseased region in the physiological image, the diseased region including the at least one mutated object.

Exemplarily, the region segmentation subnetwork includes, but not limited to, at least one of: a CNN, an LSTM, an RNN, an FCN, a U-Net, a SegNet and a LinkNet.

Exemplarily, the diseased region is used for indicating the position of the at least one mutated object. The diseased region may be indicated by marking an envelope of the diseased region in the physiological image, or by a segmentation result of the physiological image.

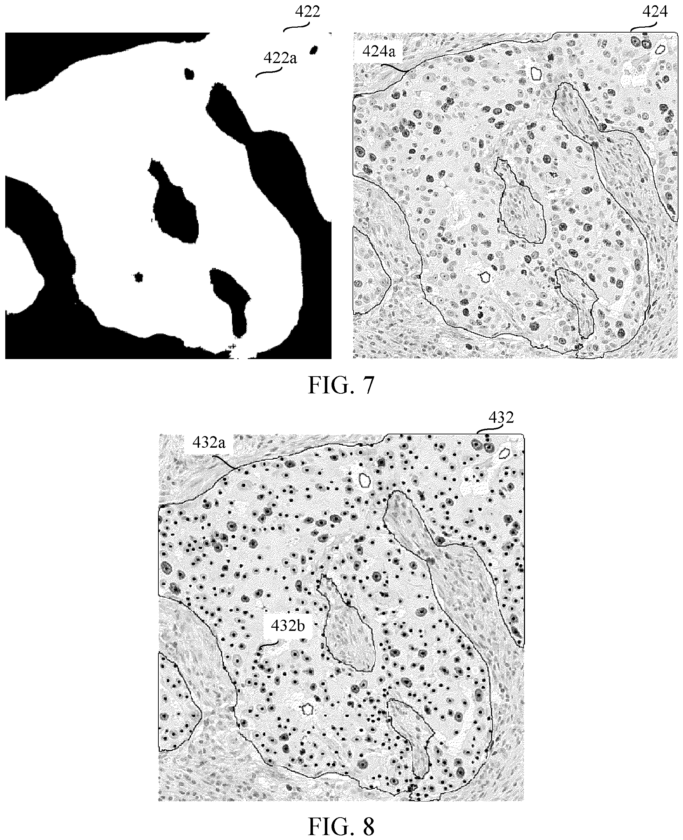

FIG. 7 shows a schematic diagram of a segmentation image and a diseased region mark image of the physiological image. In FIG. 7, the segmentation image 422 of the physiological image indicates the diseased region by the segmentation result of the physiological image, and a first region 422a in the segmentation image 422 of the physiological image is the diseased region. The color of the first regions 422a is white. Exemplarily, the first regions 422a are not a connected region, and all the white regions in the segmentation image 422 of the physiological image are the first regions 422a. The diseased region mark image 424 is generated by marking envelopes 424a of the diseased regions in the physiological image. The region enclosed by the envelope 424a of the diseased region is the diseased region.

**Step 526:** Determine the physiological objects belonging to the diseased region as the mutated objects according to the diseased region and the position information of the physiological objects, and determine the position information of the mutated objects.

Exemplarily, the physiological objects in the diseased region are determined as the mutated objects, and the position information of the mutated objects is determined according to the position information of the physiological objects. FIG. 8 shows a schematic diagram of a position mark image of mutated objects. A marked point 432b in the position mark image 432 of the mutated objects indicates a position of a mutated object. There are a plurality of marked points in the position mark image 432 of the mutated objects that respectively indicate the positions of the plurality of mutated objects.

An envelope 432a of the diseased region is also marked in the position mark image 432 of the mutated objects.

Based on the above, according to the method provided by this embodiment, the physiological image processing method is executed based on the physiological image processing model, and the detection network includes the position detection subnetwork and the region segmentation subnetwork. The position detection subnetwork and the region segmentation subnetwork are called to respectively perform the position detection and the image segmentation on the physiological image to respectively determine the position information of the physiological objects and the diseased region, so that generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

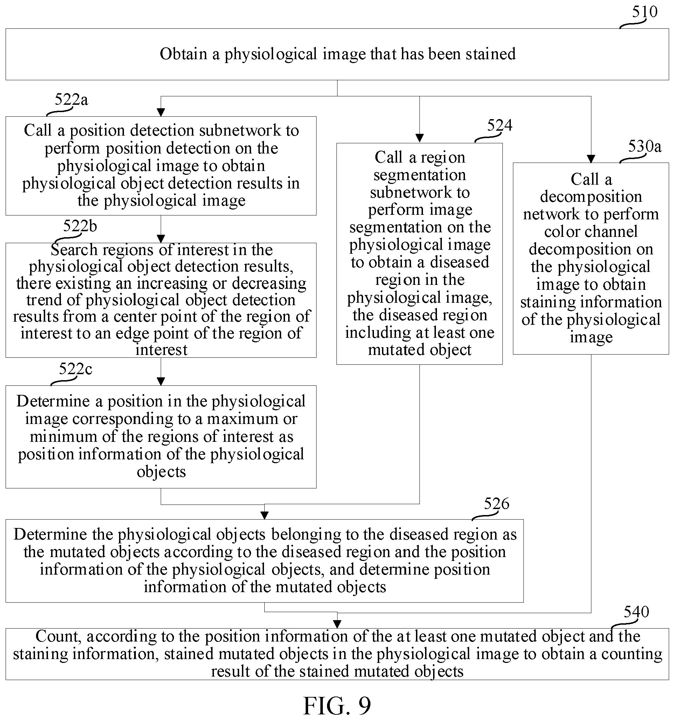

FIG. 9 shows a flow chart of a physiological image processing method provided by some exemplary embodiments of the present disclosure. The method may be executed by a computer device. That is, in the embodiment shown in FIG. 5, step 522 may be implemented as step 522a, step 522b and step 522c:

**Step 522a:** Call the position detection subnetwork to perform the position detection on the physiological image to obtain physiological object detection results of the physiological image.

Exemplarily, the physiological object detection result is used for indicating probability information that pixels in the physiological image belong to the physiological objects. Exemplarily, there is a correlation between the physiological object detection result corresponding to the pixels and the probability that the pixels belong to the physiological objects. This correlation may be a positive correlation or a negative correlation.

**Step 522b:** Search regions of interest in the physiological object detection results, there existing an increasing or decreasing trend of physiological object detection results from a center point of the region of interest to an edge point of the region of interest.

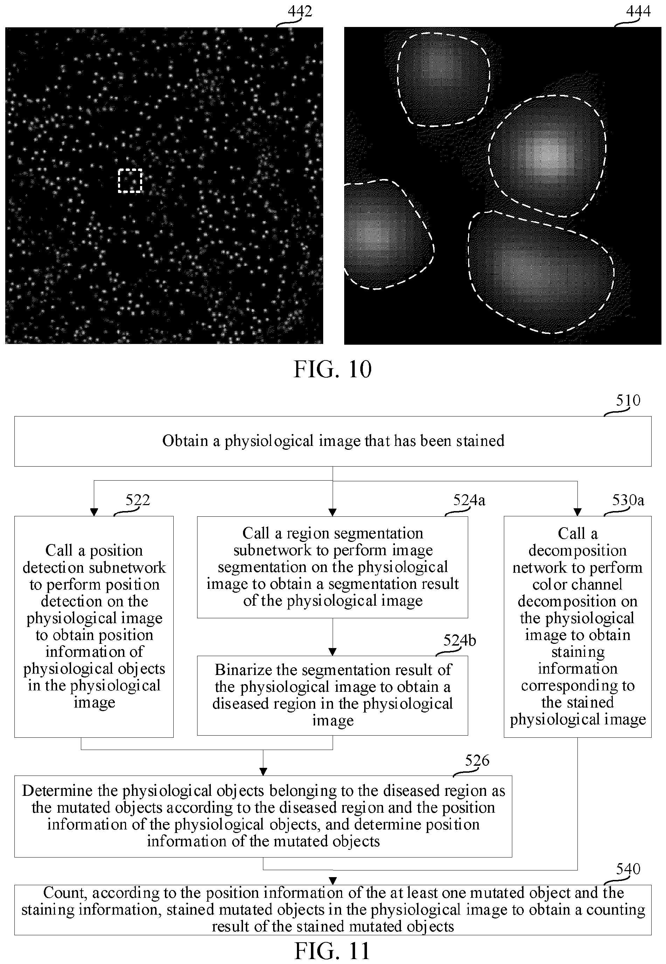

Exemplarily, the region of interest may be rectangular, circular, elliptical or in another irregular shape. The trend existing from the center point of the region of interest to the edge point of the region of interest may be a trend in one direction, or a trend in a plurality of directions. FIG. 10 shows a schematic diagram of the physiological object detection result. FIG. 10 is illustrated in an example in which there is a positive correlation between the physiological object detection result corresponding to the pixels and the probability that the pixels belong to the physiological objects. A first image 442 shows the physiological object detection result of the physiological image. In order to describe the physiological object detection result more clearly, a second image 444, which is a partial image of the first image 442, is described exemplarily. Exemplarily, the second image 444 is the cropped region in the first image 442, and in the first image 442, the cropped region is marked with a dashed line.

In the second image 444, four regions of interest are found. The regions of interest are marked with dashed lines in the second image 444. The center point of the region of interest is a first pixel, and the edge point of the region of interest is a second pixel. There is an increasing or decreasing trend, for example, a decreasing trend, in terms of the physiological object detection result from the first pixel to the second pixel.

**Step 522c:** Determine a position in the physiological image corresponding to a maximum or minimum of the regions of interest as the position information of the physiological objects.

Exemplarily, the position of the pixel corresponding to the maximum or minimum physiological object detection result in the region of interest is determined as the position information of the physiological object. In an example, the position information of the physiological object is the center position of the physiological object.

Based on the above, according to the method provided by this embodiment, the position detection subnetwork is called to perform the position detection on the physiological image so as to determine the position information of the physiological objects. The segmentation capability of the position detection subnetwork for the physiological image is fully exploited to determine the position information of the physiological objects in the physiological object detection result, and the segmentation capability of the position detection subnetwork for the physiological image is utilized to determine the position information of the physiological object. Generalization ability of the physiological image processing method is improved through the detection network, and the physiological image processing method can obtain a good counting effect on the physiological image obtained by different staining manners, thereby avoiding influence of different staining manners on the physiological image and ensuring accuracy of the counting result of the stained mutated objects.

FIG. 11 shows a flow chart of a physiological image processing method provided by some exemplary embodiments of the present disclosure. The method may be executed by a computer device. That is, in the embodiment shown in FIG. 5, step 524 may be implemented as step 524a and step 524b:

**Step 524a:** Call the region segmentation subnetwork to perform image segmentation on the physiological image to obtain a segmentation result of the physiological image.

Exemplarily, the segmentation result is used for indicating probability information that a plurality of pixels in the physiological image belong to the diseased region. Exemplarily, the segmentation result of the physiological image is a grayscale image, and there is a positive correlation between the grayscale value corresponding to a pixel in the grayscale image and the probability that the pixel belongs to the diseased region.

**Step 524b:** Binarize the segmentation result of the physiological image to obtain the diseased region in the physiological image.

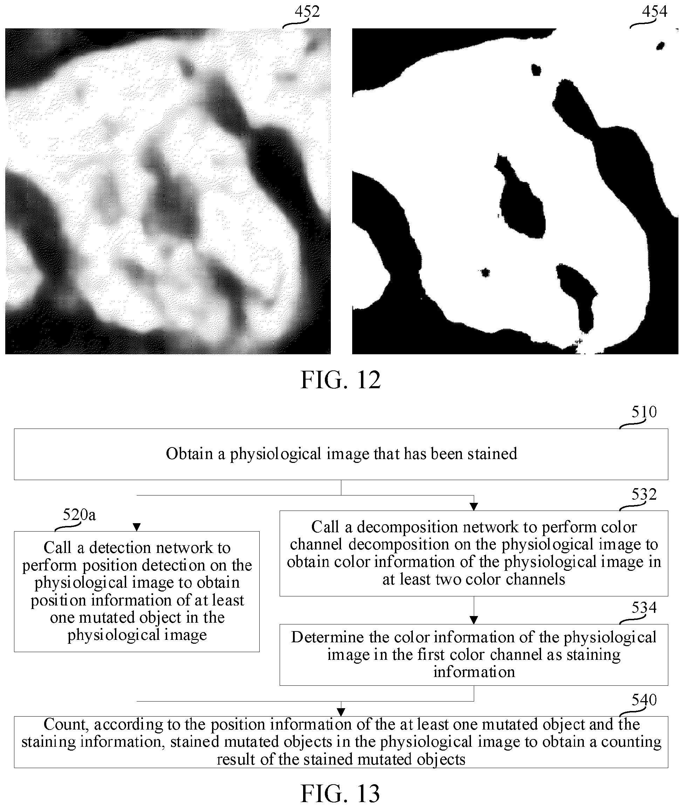

Exemplarily, the segmentation result of the physiological image is binarized to divide the physiological image into diseased regions and non-diseased regions. Exemplarily, the segmentation result of the physiological image is a grayscale image, and the grayscale image is binarized to obtain the diseased regions in the physiological image. FIG. 12 shows a schematic diagram of a segmentation result image and a diseased region mark image of the physiological image. The segmentation result image 452 of the physiological image is obtained by calling the region segmentation subnetwork to perform image segmentation on the physiological image. The segmentation result image 452 of the physiological image is binarized to obtain a diseased region image 454.

Based on the above, according to the method provided by this embodiment, the region segmentation subnetwork is called to perform the image segmentation on the physiological image so as to determine the diseased region in the physiological image. The segmentation capability of the region segmentation subnetwork for the physiological image is fully exploited to determine the diseased region in the segmentation result of the physiological image, and the segmentation capability of the region segmentation subnetwork for the physiological image is utilized to determine the diseased region. Generalization ability of the physiological image processing method is improved through the detection network, and the physiological image processing method can obtain a good counting effect on the physiological image obtained by different staining manners, thereby avoiding influence of different staining manners on the physiological image and ensuring accuracy of the counting result of the stained mutated objects.

FIG. 13 shows a flow chart of a physiological image processing method provided by some exemplary embodiments of the present disclosure. The method may be executed by a computer device. That is, in the embodiment shown in FIG. 4, step 530a may be implemented as step 532 and step 534:

**Step 532:** Call the decomposition network to perform the color channel decomposition on the physiological image to obtain color information of the physiological image in at least two color channels.

Exemplarily, the color channels are channels that store color information of the physiological image, and the color information of the physiological image may be colors of the physiological image, such as red, blue, brown, yellow and the like, or color attributes of the physiological image, such as saturation, hue, brightness and the like. Exemplarily, in the color information of the physiological image obtained by the color channel decomposition in at least two color channels, the at least two color channels belong to the same color system. For example, the color system is at least one of a red green blue (RGB) color system, a hue saturation value (HSV) color system and a hematoxylin-eosin staining-diaminobezidin 3 (DAB) (HED) color system (also referred to as a hematoxylin eosin 3-diaminobenzidine color system).

**Step 534:** Determine the color information of the physiological image in the first color channel as the staining information.

The staining information is used for indicating staining statuses of the physiological objects in the stained physiological image, i.e., the staining information is obtained based on the stained physiological image.

Exemplarily, the staining information is determined based on the color information or color attributes in the color channels. In the color information of the physiological image in the at least two color channels, the color information in the first color channel is determined as the staining information. Exemplarily, the color information of the physiological image in the DAB color channel is determined as the staining information. Exemplarily, the staining information is used for indicating the staining statuses of the physiological objects in the physiological image. For example, different staining manners have different staining effects on the physiological objects in the physiological image. The staining information is used for indicating the staining effects of the physiological objects in the physiological image.

In some embodiments, the staining effect refers to the color difference of the physiological image. For example, the color of the mutated object is red, and the color of the physiological object is blue.

In some embodiments, the staining effect refers to the difference between color attribute values of the physiological image. For example, the saturation of the mutated object is 5, and the saturation of the physiological object is 3.

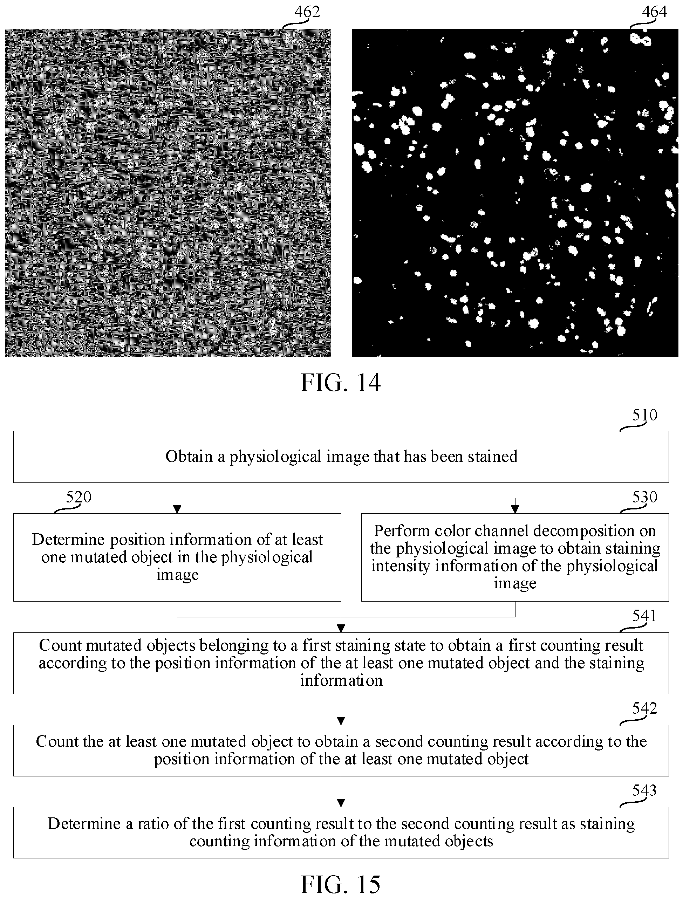

FIG. 14 shows a schematic diagram of staining information. Exemplarily, the first image 462 is the color information of the physiological image in the DAB color channel in the hematoxylin-eosin staining-DAB color system. The color information of the stained physiological image in the DAB color channel is determined as the staining information. In an exemplary implementation, the staining information is binarized to obtain a second image 464. The second image 464 is the staining information obtained after processing.

In some embodiments, the computer device calls the decomposition network to perform the color channel decomposition on each pixel in the physiological image, such that each pixel is decomposed into at least two color channels to obtain the color information of each pixel in the at least two color channels. In the color information of the pixel corresponding to the at least two color channels, the computer device determines the color information in the first color channel as pixel staining information of the pixel.

The computer device determines the staining information corresponding to the physiological image based on the pixel staining information corresponding to each pixel in the physiological image.

Based on the above, according to the method provided by this embodiment, the physiological image processing method is executed based on the physiological image processing model, and the decomposition network is called to perform the color channel decomposition on the physiological image so as to determine the staining information of the mutated objects. The color information carried in the sample image is fully extracted, and the color information is utilized to indicate the staining statuses of the stained physiological objects in the sample image. Generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

FIG. 15 shows a flow chart of a physiological image processing method provided by some exemplary embodiments of the present disclosure. The method may be executed by a computer device. That is, in the embodiment shown in FIG. 3, step 540 may be implemented as step 541, step 542 and step 543:

**Step 541:** Count mutated objects belonging to a first staining state to obtain a first counting result according to the position information of the at least one mutated object and the staining information.

In an implementation, the staining counting result includes staining counting information. The staining counting information is used for indicating a ratio of the number of the mutated objects in the physiological image to the number of all the mutated objects. In another implementation, the staining counting information may also be used for indicating a counting result of the mutated objects in the first staining state, for example, a ratio of the number of the mutated objects in the colored state to the number of all the mutated objects, or a ratio of the number of the mutated objects in the uncolored state to the number of all the mutated objects.

The staining information is used for indicating the staining statuses of the physiological objects in the physiological image after the color channel decomposition. For example, the physiological objects in the physiological image treated by different staining manners have different staining statuses, or in the same staining manner, the physiological objects and the mutated objects have different staining status; or the staining information is used for indicating staining states of the physiological objects in the physiological image after color channel decomposition. Specifically, the staining information is used for indicating at least two staining states of the physiological objects in the physiological image after color channel decomposition.

The staining information is obtained in the physiological image after color channel decomposition.

In an implementation, in a case that the staining information is determined based on the color attributes in the color channel, a region of the physiological image with the staining information exceeding a staining threshold is determined as a first staining region. The first staining region corresponds the physiological objects belonging to the first staining state. Exemplarily, the first staining state is used for indicating whether the physiological object belongs to a colored state or an uncolored state.

The staining threshold refers to the threshold corresponding to the values of the color attribute in the color channel. Taking the saturation as an example, the staining threshold of the saturation is set to 3, and the region in the physiological image with the saturation exceeding 3 is determined as the first staining region.

The mutated objects belonging to the first staining state are determined according to the first staining region and the position information of the mutated objects. Exemplarily, the mutated objects located in the first staining region are determined as the mutated objects belonging to the first staining state.

The mutated objects belonging to the first staining state are counted to obtain the first counting result. The number of the mutated objects belonging to the first staining state is counted as the first counting result.

**Step 542:** Count the at least one mutated object to obtain a second counting result according to the position information of the at least one mutated object.

A statistical treatment is performed on the mutated objects in the physiological image according to the position information of the mutated objects to obtain the second counting result of the mutated objects.

**Step 543:** Determine a ratio of the first counting result to the second counting result as the staining counting information of the mutated objects.

The staining counting information of the mutated objects is used for indicating the ratio of the first counting result to the second counting result. Exemplarily, the staining counting information is used for indicating the ratio of the number of the mutated objects in the first staining state to the number of all the mutated objects.

Based on the above, according to the method provided by this embodiment, the staining counting result includes the staining counting information, and the mutated objects are counted to obtain the staining counting information of the mutated objects. The counting result of the stained mutated objects in the physiological image is quickly obtained. The staining states of the mutated objects are described using the staining counting information. Generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

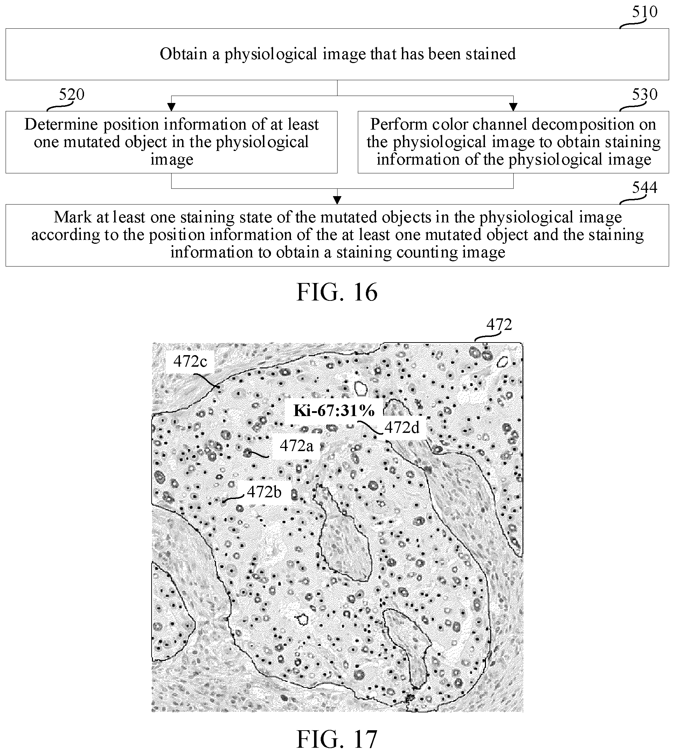

FIG. 16 shows a flow chart of a physiological image processing method provided by some exemplary embodiments of the present disclosure. The method may be executed by a computer device. That is, in the embodiment shown in FIG. 3, step 540 may be implemented as step 544:

**Step 544:** Mark at least one staining state of the mutated objects in the physiological image according to the position information and the staining information to obtain the staining counting image.

In an implementation, the staining counting result includes the staining counting image. At least one staining state of the mutated objects is marked in the physiological image obtain the staining counting image.

In the staining counting image, the mutated objects in only one staining state may be marked; or the mutated objects in multiple staining states may be marked. In an exemplary implementation, envelopes of the diseased regions may also be marked in the staining counting image.

Exemplarily, FIG. 17 shows a schematic diagram of a staining counting image. Exemplarily, in the staining counting image 472, the mutated object belonging to the colored state is marked by a first marked point 472a, and the mutated object belonging to the uncolored state is marked by a second marked point 472b. Exemplarily, the staining counting image 472 further includes an envelope 472c of the diseased region, and a ratio 472d of the number of the mutated objects belonging to the colored state to the number of all the mutated objects. Ki-67 is used for indicating the staining manner for the physiological image, and 31% is used for indicating the ratio of the number of the mutated objects belonging to the colored state to the number of all the mutated objects.

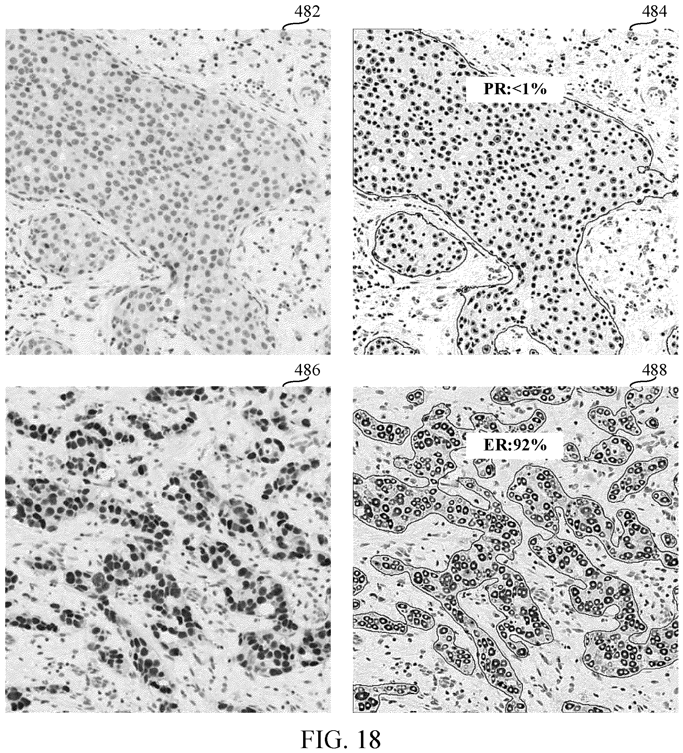

Exemplarily, FIG. 18 shows a schematic diagram of physiological images and staining counting images. The first physiological image 482 is a physiological image subjected to PR staining, and the first staining counting image 484 is a staining counting image corresponding to the first physiological image 482. The second physiological image 486 is a physiological image subjected to ER staining, and the second staining counting image 488 is a staining counting image corresponding to the second physiological image 486.

Based on the above, according to the method provided by this embodiment, the staining counting result includes the staining counting image, and the staining states of the mutated objects are marked in the physiological image to obtain the staining counting image of the mutated objects. The counting result of the stained mutated objects in the physiological image is quickly obtained. The staining states of the mutated objects are described using the staining counting image. Generalization ability of the physiological image processing method is improved, and the physiological image processing method can obtain a good counting effect on the physiological image obtained by different staining manners, thereby avoiding influence of different staining manners on the physiological image and ensuring accuracy of the counting result of the stained mutated objects.

Next, a training method of a physiological image processing model will be described through the following embodiments.

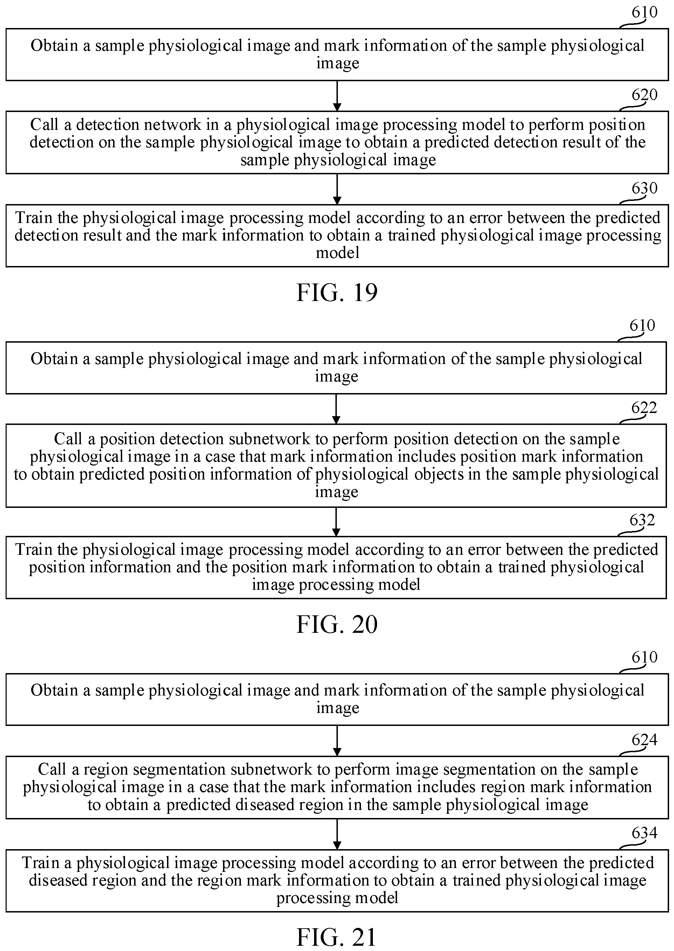

FIG. 19 shows a flow chart of a training method of a physiological image processing model provided by some exemplary embodiments of the present disclosure. The method may be executed by a computer device. The method includes:

**Step 610:** Obtain a sample physiological image and mark information of the sample physiological image.

Exemplarily, the sample physiological image is a marked physiological image. The mark information of the sample physiological image is used for indicating a marking result of the sample physiological image. In this embodiment, the mark information of the sample physiological image may be the position information of the mutated objects in the sample physiological image, or other information related to the position information of the mutated objects. This embodiment does not limit the specific contents of the mark information. In an example, the mark information of the sample physiological image is information related to the position information of the mutated objects.

In an exemplary implementation, the mark information of the sample physiological image includes: position mark information and/or region mark information. The position mark information is used for indicating the position information of the physiological objects in the sample physiological image, and the region mark information is used for indicating the diseased regions in the sample physiological image.

**Step 620:** Call a detection network in the physiological image processing model to perform position detection on the sample physiological image to obtain a predicted detection result of the sample physiological image.

Exemplarily, the physiological image processing model includes the detection network. The detection network is configured to perform the position detection on the sample physiological image to obtain the predicted detection result of the sample physiological image. In an example, the predicted detection result of the sample physiological image is predicted position information of the mutated objects.

In an exemplary implementation, the physiological image processing model further includes a decomposition network. The decomposition network is configured to perform color channel decomposition on the sample physiological image to obtain predicted staining information. In an example, the decomposition network determines the predicted staining information by a statistical method. There are no network parameters that need to be trained in the decomposition network. The decomposition network in this embodiment is the same as the decomposition network of the trained physiological image processing model. In another exemplary implementation, only the detection network in the physiological image processing model is trained, and the decomposition network is added to the trained physiological image processing model, so as to construct the physiological image processing model in the embodiment of the physiological image processing method above.

**Step 630:** Train the physiological image processing model according to an error between the predicted detection result and the mark information to obtain a trained physiological image processing model.

Exemplarily, a prediction error is obtained through the difference between the predicted detection result and the mark information. The physiological image processing model is trained through the prediction error to obtain the physiological image processing model in any of the embodiments above.

Exemplarily, a back propagation algorithm is utilized to update parameters of the physiological image processing model, and errors obtained based on a plurality of sample physiological images and mark information of the plurality of sample physiological images are utilized to update the parameters of the physiological image processing model so as to improve prediction accuracy of the physiological image processing model.

Based on the above, according to the method provided by this embodiment, the physiological image processing model is trained according to the error between the predicted detection result and the mark information to improve the prediction accuracy of the physiological image processing model, thereby laying a foundation for calling the physiological image processing model to process the physiological image. The position detection is performed on the sample physiological image through the detection network to obtain the predicted detection result of the sample physiological image, so that generalization ability of the physiological image processing model is improved, and accuracy of the counting result of the stained mutated objects is ensured.

FIG. 20 shows a flow chart of a training method of a physiological image processing model provided by some exemplary embodiments of the present disclosure. The method may be executed by a computer device. That is, in the embodiment shown in FIG. 19, step 620 may be implemented as step 622, and step 630 may be implemented as step 632:

**Step 622:** Call a position detection subnetwork to perform the position detection on the sample physiological image in a case that mark information includes position mark information to obtain predicted position information of the physiological objects in the sample physiological image.

Exemplarily, the mark information includes the position mark information, and the position mark information is used for indicating position information of the physiological objects in the sample physiological image. The detection network includes the position detection subnetwork. This embodiment does not impose any restrictions on whether the detection network includes other subnetworks. In an implementation, the detection network further includes a region segmentation subnetwork. Training process of the region segmentation subnetwork and the position detection subnetwork in the detection network may be independent of each other. Exemplarily, the position mark information is obtained after marking. In an implementation, the size of the sample physiological image is 2000*2000 pixels.

**Step 632:** Train the physiological image processing model according to an error between the predicted position information and the position mark information to obtain a trained physiological image processing model.

Exemplarily, the initial physiological image processing model is the detection network trained through a public dataset (Image Net dataset), which is trained through a public dataset Image Net dataset to obtain the trained physiological image processing model. In an example, the image size of the Image Net dataset is 512*512 pixels, the batch size is 8, the learning rate is 0.0001, and the maximum number of iterations is 200.

Based on the above, according to the method provided by this embodiment, the physiological image processing model is trained according to the error between the predicted position information and the position mark information to improve the prediction accuracy of the physiological image processing model, thereby laying a foundation for calling the physiological image processing model to process the physiological image. The position detection is performed on the sample physiological image through the detection network to obtain the predicted detection result of the sample physiological image, so that generalization ability of the physiological image processing model is improved, and accuracy of the counting result of the stained mutated objects is ensured.

FIG. 21 shows a flow chart of a training method of a physiological image processing model provided by some exemplary embodiments of the present disclosure. The method may be executed by a computer device. That is, in the embodiment shown in FIG. 19, step 620 may be implemented as step 624, and step 630 may be implemented as step 634:

**Step 624:** Call the region segmentation subnetwork to perform image segmentation on the sample physiological image in a case that the mark information includes the region mark information to obtain a predicted diseased region in the sample physiological image.

Exemplarily, the predicted diseased region includes at least one mutated object.

Exemplarily, the mark information includes the region mark information, and the region mark information is used for indicating the diseased region in the sample physiological image. The detection network includes the region segmentation subnetwork. This embodiment does not impose any restrictions on whether the detection network includes other subnetworks. In an implementation, the detection network further includes a position detection subnetwork. Training process of the region segmentation subnetwork and the position detection subnetwork in the detection network may be independent of each other. Exemplarily, the position mark information is obtained after marking. In an implementation, the sample physiological image is subjected to data augmentation. The data augmentation includes, but not limited to, at least one of random flipping, random cropping and stain color perturbation.

**Step 634:** Train the physiological image processing model according to an error between the predicted diseased region and the region mark information to obtain a trained physiological image processing model.

Exemplarily, the initial physiological image processing model is the detection network trained through a public dataset (Image Net dataset), which is trained through a public dataset (Image Net dataset) to obtain the trained physiological image processing model.

Exemplarily, the error between the predicted diseased region and the region mark information includes: at least one of a mean square error (MSE) loss function, a cross-entropy loss function and a mean absolute error (MAE) loss function.

Based on the above, according to the method provided by this embodiment, the physiological image processing model is trained according to the error between the predicted diseased region and the region mark information to improve the prediction accuracy of the physiological image processing model, thereby laying a foundation for calling the physiological image processing model to process the physiological image. The position detection is performed on the sample physiological image through the detection network to obtain the predicted detection result of the sample physiological image, so that generalization ability of the physiological image processing model is improved, and accuracy of the counting result of the stained mutated objects is ensured.

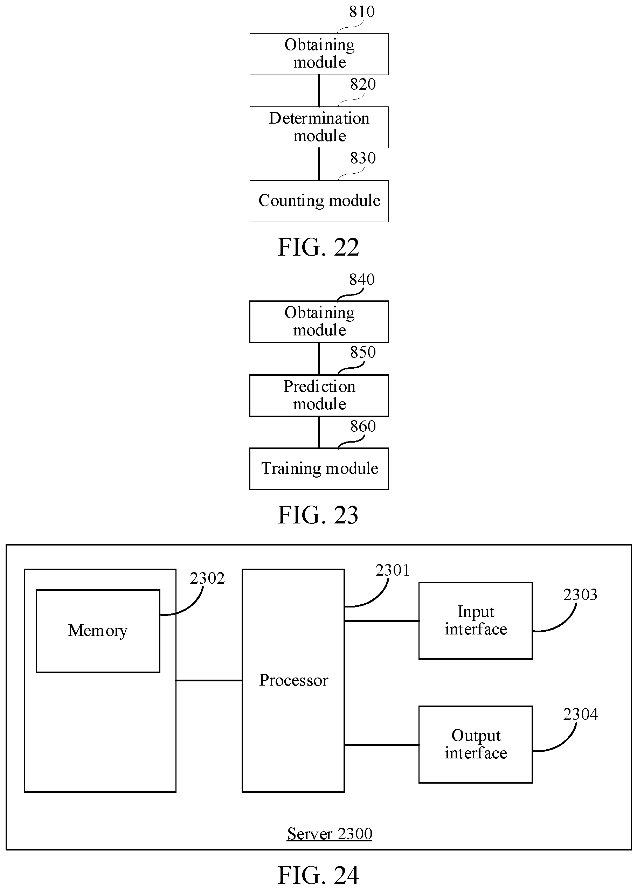

FIG. 22 shows a block diagram of a physiological image processing apparatus provided by some exemplary embodiments of the present disclosure. The apparatus includes:
an obtaining module 810, configured to execute step 510 in the embodiment of FIG. 3;
a determination module 820, configured to execute step 520 in the embodiment of FIG. 3,
the determination module 820 being further configured to execute step 530 in the embodiment of FIG. 3; and
a counting module 830, configured to execute step 540 in the embodiment of FIG. 3.

In an exemplary design of the present disclosure, the determination module 820 is further configured to execute step 520a and step 530a in the embodiment of FIG. 4.

In an exemplary design of the present disclosure, the determination module 820 is further configured to execute step 522, step 524 and step 526 in the embodiment of FIG. 5.

In an exemplary design of the present disclosure, the determination module 820 is further configured to execute step 522a, step 522b and step 522c in the embodiment of FIG. 9.

In an exemplary design of the present disclosure, the determination module 820 is further configured to execute step 524a and step 524b in the embodiment of FIG. 11.

In an exemplary design of the present disclosure, the determination module 820 is further configured to execute step 532 and step 534 in the embodiment of FIG. 13.

In an exemplary design of the present disclosure, the counting module 830 is further configured to execute step 541, step 542 and step 543 in the embodiment of FIG. 15.

In an exemplary design of the present disclosure, the counting module 830 is further configured to execute step 544 in the embodiment of FIG. 16.

FIG. 23 shows a block diagram of a training apparatus of a physiological image processing model provided by some exemplary embodiments of the present disclosure. The apparatus includes:
an obtaining module 840, configured to execute step 610 in the embodiment of FIG. 19;
a prediction module 850, configured to execute step 620 in the embodiment of FIG. 19; and
a training module 860, configured to execute step 630 in the embodiment of FIG. 19.

The prediction module 850 is further configured to execute step 622 in the embodiment of FIG. 20. The training module 860 is further configured to execute step 632 in the embodiment of FIG. 20.

The prediction module 850 is further configured to execute step 624 in the embodiment of FIG. 21. The training module 860 is further configured to execute step 634 in the embodiment of FIG. 21.

An embodiment of the present disclosure further provides a computer device, including: a processor and a memory. The memory stores at least one program, and the processor is configured to execute the at least one program in the memory to implement the physiological image processing method and/or the training method of a physiological image processing model provided in the method embodiments above.

In some embodiments, the computer device is a server. Exemplarily, FIG. 24 is a structural block diagram of a server provided by some exemplary embodiments of the present disclosure.

Usually, the server 2300 includes: a processor 2301 and a memory 2302.

The processor 2301 may include one or more processing cores, for example, a 4-core processor or an 8-core processor. The processor 2301 may be implemented in at least one hardware form of a digital signal processor (DSP), a field-programmable gate array (FPGA), and a programmable logic array (PLA). The processor 2301 may also include a main processor and a coprocessor. The main processor is a processor configured to process data in an awake state, and is also referred to as a central processing unit (CPU). The coprocessor is a low power consumption processor configured to process the data in a standby state. In some embodiments, the processor 2301 may be integrated with a graphics processing unit (GPU). The GPU is configured to render and draw contents that need to be displayed on a display screen. In some embodiments, the processor 2301 may further include an artificial intelligence (AI) processor. The AI processor is configured to process computing operations related to machine learning.

The memory 2302 may include one or more computer-readable storage media. The computer-readable storage medium may be non-transient. The memory 2302 may further include a high-speed random access memory and a nonvolatile memory, for example, one or more disk storage devices or flash storage devices. In some embodiments, the non-transitory computer-readable storage medium in the memory 2302 is configured to store at least one instruction, and the at least one instruction is configured to be executed by the processor 2301 to implement the physiological image processing method and/or the training method of a physiological image processing model provided in the method embodiments of the present disclosure.

In some embodiments, the server 2300 may further include: an input interface 2303 and an output interface 2304. The processor 2301, the memory 2302, the input interface 2303 and the output interface 2304 may be connected through buses or signal cables. Each peripheral device may be connected to the input interface 2303 and the output interface 2304 through a bus, a signal cable, or a circuit board. The input interface 2303 and the output interface 2304 may be configured to connect the at least one peripheral device related to input/output (I/O) to the processor 2301 and the memory 2302. In some embodiments, the processor 2301, the memory 2302, the input interface 2303 and the output interface 2304 are integrated on the same chip or circuit board. In other embodiments, any one or two of the processor 2301, the memory 2302, the input interface 2303 and the output interface 2304 may be implemented on a single chip or circuit board, which is not limited in this embodiment of the present disclosure.

A person skilled in the art may understand that the structures shown above constitute no limitation on the server 2300, and the server may include more or fewer components than those shown in the figures, or some components may be combined, or a different component deployment may be used.

An exemplary embodiment further provides a chip, including a programmable logic circuit and/or program instructions. The chip, when run on a computer device, is configured to implement the physiological image processing method and/or the training method of a physiological image processing model in the above aspects.

An exemplary embodiment further provides a computer program product. The computer program product includes computer instructions, and the computer instructions are stored in a computer-readable storage medium. A processor of a computer device reads the computer instructions from the computer-readable storage medium. The processor reads and executes the computer instructions from the computer device to implement the physiological image processing method and/or the training method of a physiological image processing model provided in the method embodiments above.

An exemplary embodiment further provides a computer-readable storage medium. The computer-readable storage medium stores a computer program. The computer program is loaded and executed by a processor to implement the physiological image processing method and/or the training method of a physiological image processing model provided in the method embodiments above.

## Claims

1. A physiological image processing method, executable by a computer device, the method comprising:
obtaining a physiological image that has been stained;
determining position information of at least one mutated object in the physiological image;
performing color channel decomposition on the physiological image to obtain staining information corresponding to the physiological image; and
counting, according to the position information of the at least one mutated object and the staining information, stained mutated objects in the physiological image to obtain a counting result of the stained mutated objects.

2. The method according to claim 1, wherein the method is executable based on a physiological image processing model in a computer device, and the physiological image processing model comprises a detection network and a decomposition network; and
the determining position information of at least one mutated object in the physiological image comprises:
calling the detection network to perform position detection on the physiological image to obtain the position information of the at least one mutated object in the physiological image; and
the performing color channel decomposition on the physiological image to obtain staining information corresponding to the physiological image comprises:
calling the decomposition network to perform the color channel decomposition on the physiological image to obtain the staining information corresponding to the physiological image.

3. The method according to claim 2, wherein the detection network comprises a position detection subnetwork and a region segmentation subnetwork; and
the calling the detection network to perform position detection on the physiological image to obtain the position information of the at least one mutated object in the physiological image comprises:
calling the position detection subnetwork to perform the position detection on the physiological image to obtain position information of physiological objects in the physiological image;
calling the region segmentation subnetwork to perform image segmentation on the physiological image to obtain a diseased region in the physiological image, the diseased region comprising the at least one mutated object; and
determining physiological objects belonging to the diseased region as the at least one mutated object according to the diseased region and the position information of the physiological objects, and determining the position information of the at least one mutated object.

4. The method according to claim 3, wherein the calling the position detection subnetwork to perform the position detection on the physiological image to obtain position information of physiological objects in the physiological image comprises:
calling the position detection subnetwork to perform the position detection on the physiological image to obtain physiological object detection results of the physiological image;
searching regions of interest in the physiological object detection results, there existing an increasing or decreasing trend of physiological object detection results from a center point of the region of interest to an edge point of the region of interest; and
determining a position in the physiological image corresponding to a maximum or minimum of the regions of interest as the position information of the physiological objects.

5. The method according to claim 3 or 4, wherein the calling the region segmentation subnetwork to perform image segmentation on the physiological image to obtain a diseased region in the physiological image comprises:
calling the region segmentation subnetwork to perform the image segmentation on the physiological image to obtain a segmentation result of the physiological image, the segmentation result being used for indicating probability information that a plurality of pixels of the physiological image belong to the diseased region; and
binarizing the segmentation result of the physiological image to obtain the diseased region in the physiological image.

6. The method according to any one of claims 2 to 5, wherein the calling the decomposition network to perform the color channel decomposition on the physiological image to obtain the staining information corresponding to the physiological image comprises:
calling the decomposition network to perform the color channel decomposition on the physiological image to obtain color information of the physiological image in at least two color channels; and
determining the color information of the physiological image in the first color channel as the staining information.

7. The method according to any one of claims 1 to 6, wherein the staining counting result comprises staining counting information; and
the counting, according to the position information of the at least one mutated object and the staining information, stained mutated objects in the physiological image to obtain a counting result of the stained mutated objects comprises:
counting, according to the position information of the at least one mutated object and the staining information, mutated objects belonging to a first staining state to obtain a first counting result, the staining information indicating at least two staining states of physiological objects in the physiological image;
counting, according to the position information of the at least one mutated object, the at least one mutated object to obtain a second counting result; and
determining a ratio of the first counting result to the second counting result as the staining counting information of the mutated objects.

8. The method according to claim 7, wherein the counting, according to the position information of the at least one mutated object and the staining information, mutated objects belonging to a first staining state to obtain a first counting result comprises:
determining a region of the physiological image with staining information of the region exceeding a staining threshold as a first staining region, the first staining region corresponding to the physiological objects belonging to the first staining state;
determining the mutated objects belonging to the first staining state according to the first staining region and the position information of the at least one mutated object; and
counting the mutated objects belonging to the first staining state to obtain the first counting result.

9. The method according to any one of claims 1 to 6, wherein the staining counting result comprises a staining counting image; and
the counting, according to the position information of the at least one mutated object and the staining information, stained mutated objects in the physiological image to obtain a counting result of the stained mutated objects comprises:
marking at least one staining state of the mutated objects in the physiological image according to the position information of the at least one mutated object and the staining information to obtain the staining counting image.

10. A training method of a physiological image processing model, executable by a computer device, the physiological image processing model comprising a detection network, and the method comprising:
obtaining a sample physiological image and mark information of the sample physiological image;
calling the detection network in the physiological image processing model to perform position detection on the sample physiological image to obtain a predicted detection result of the sample physiological image; and
training the physiological image processing model according to an error between the predicted detection result and the mark information to obtain a trained physiological image processing model.

11. The method according to claim 10, wherein the mark information of the sample physiological image comprises: position mark information and/or region mark information;
wherein the position mark information is used for indicating position information of physiological objects in the sample physiological image, and the region mark information is used for indicating a diseased region in the sample physiological image.

12. The method according to claim 10 or 11, wherein the detection network comprises a position detection subnetwork;
the calling the detection network in the physiological image processing model to perform position detection on the sample physiological image to obtain a predicted detection result of the sample physiological image comprises:
calling the position detection subnetwork to perform the position detection on the sample physiological image in a case that mark information comprises the position mark information to obtain predicted position information of the physiological objects in the sample physiological image; and
the training the physiological image processing model according to an error between the predicted detection result and the mark information to obtain a trained physiological image processing model comprises:
training the physiological image processing model according to the predicted position information and the position mark information to obtain the trained physiological image processing model.

13. The method according to claim 10 or 11, wherein the detection network comprises a region segmentation subnetwork;
the calling the detection network in the physiological image processing model to perform position detection on the sample physiological image to obtain a predicted detection result of the sample physiological image comprises:
calling the region segmentation subnetwork to perform image segmentation on the sample physiological image in a case that the mark information comprises the region mark information to obtain a predicted diseased region in the sample physiological image, the predicted diseased region comprising at least one mutated object; and
the training the physiological image processing model according to an error between the predicted detection result and the mark information to obtain a trained physiological image processing model comprises:
training the physiological image processing model according to the predicted diseased region and the region mark information to obtain the trained physiological image processing model.

14. A physiological image processing apparatus, comprising:
an obtaining module, configured to obtain a physiological image that has been stained;
a determination module, configured to determine position information of at least one mutated object in the physiological image,
the determination module being further configured to perform color channel decomposition on the physiological image to obtain staining information corresponding to the physiological image; and
a counting module, configured to count, according to the position information of the at least one mutated object and the staining information, stained mutated objects in the physiological image to obtain a counting result of the stained mutated objects.

15. A training apparatus of a physiological image processing model, the physiological image processing model comprising a detection network, and the apparatus comprising:
an obtaining module, configured to obtain a sample physiological image and mark information of the sample physiological image;
a prediction module, configured to call the detection network in the physiological image processing model to perform position detection on the sample physiological image to obtain a predicted detection result of the sample physiological image; and
a training module, configured to train the physiological image processing model according to an error between the predicted detection result and the mark information to obtain a trained physiological image processing model.

16. A computer device, comprising: a processor and a memory, the memory storing at least one program, and the processor being configured to execute the at least one program in the memory to implement the physiological image processing method according to any one of claims 1 to 9 and/or the training method of a physiological image processing model according to any one of claims 10 to 13.

17. A computer-readable storage medium, the readable storage medium storing at least one program, and the at least one program being loaded and executed by a processor to implement the physiological image processing method according to any one of claims 1 to 9 and/or the training method of a physiological image processing model according to any one of claims 10 to 13.

18. A computer program product, comprising computer instructions stored in a computer-readable storage medium, a processor reading and executing the computer instructions from the computer-readable storage medium to implement the physiological image processing method according to any one of claims 1 to 9 and/or the training method of a physiological image processing model according to any one of claims 10 to 13.
